# EUROPEAN PATENT APPLICATION

(11) **EP 4 091 607 A1**
(43) Date of publication of application: **23.11.2022**
(21) Application number: 21206327.5
(22) Date of filing: 03.11.2021
(51) Int. Cl.: A61K 31/137, A61K 31/222, A61K 31/4025, A61K 31/4439, A61K 31/46, A61K 31/4725, A61K 31/496, A61K 31/519, A61K 31/551, A61K 31/5513, A61K 31/554, A61K 45/06, A61P 25/04, A61P 25/14, A61P 25/16, A61P 25/18, A61P 25/24, A61P 25/28

(54) **METHODS FOR TREATING CENTRAL NERVOUS SYSTEM DISORDERS WITH MUSCARINIC RECEPTOR ACTIVATOR XANOMELINE AND ANTIPSYCHOTICS**

(30) Priority: 18.05.2021 US 202163190136 P; 01.06.2021 US 202163195524 P
(71) Applicant: Karuna Therapeutics, Inc., Boston MA 02110 (US)
(72) Inventor: FELDER, Christian, Boston, MA Massachusetts 02110 (US)
(74) Representative: Bridle, Andrew Barry

(57) **Abstract**

Provided is a method of treating a central nervous system disorder in a patient in need thereof, the method comprising co-administering a first composition comprising xanomeline and/or a salt thereof and a second composition comprising an antipsychotic. In certain embodiments, a method of treating schizophrenia in a patient in need thereof is provided. For example, the method comprises co-administering a first composition comprising xanomeline and/or a salt thereof and a second composition comprising risperidone or aripiprazole. In certain embodiments, the first composition comprising xanomeline and/or a salt thereof further comprises an anticholinergic agent, such as a muscarinic antagonist.

## Description

The present disclosure relates to compositions and their application as pharmaceuticals for treating disorders ameliorated by activating muscarinic receptors in a human or animal subject combined with atypical antipsychotics.

Schizophrenia and various mood disorders are thought to be caused by an excess of dopaminergic D2 and serotonergic 5-HT2A activity, resulting in overactivity of central mesolimbic pathways and mesocortical pathways, respectively. All currently approved antipsychotic drugs, sometimes referred to as neuroleptics or major tranquilizers, work primarily by binding to and blocking dopamine D2 receptors. Antipsychotic medications have both a short-term sedative effect and the long-term effect of reducing the chances of psychotic episodes.

Atypical antipsychotics, such as risperidone, olanzapine, quetiapine, and aripiprazole, are currently the first-line agents in patients with psychotic symptoms of dementia. Nevertheless, these drugs exert detrimental effects and provide limited efficacy, such as excessive sedation, orthostatic hypotension, and related complications such as falls, extrapyramidal symptoms, cognitive slowing, cardiovascular complications, and anticholinergic side effects. Pertaining to the atypical antipsychotics, weight gain and metabolic dysfunction are prominent side-effects.

Xanomeline, an M1/M4 preferring muscarinic receptor agonist, has been shown to have therapeutic activity in improving both cognitive and behavioral symptoms associated with Alzheimer's Disease, schizophrenia, and dementia-related psychosis. However, peripherally-mediated muscarinic agonist adverse events prevented its further development. Recently, a co-formulation of xanomeline together with the peripherally-restricted muscarinic antagonist trospium (identified as KarXT) was reported to be well-tolerated and efficacious in treating acute psychosis in patients with schizophrenia.

Activating the muscarinic system through muscarinic agonists may treat several diseases, such as dementia-related psychosis, schizophrenia, Alzheimer's disease, Parkinson's disease, depression, movement disorders, drug addiction, pain, and other neurodegenerative disorders, such as tauopathies or synucleinopathies. Muscarinic cholinergic receptors are G-protein coupled receptors with five different receptor subtypes (M1-M5), each of which is found in the CNS with different tissue distributions. M1 and M4 subtypes have been of interest as therapeutic targets for various diseases. Genetic evidence directly links the muscarinic system to alcohol addiction and schizophrenia.

There remains a need in the art to treat central nervous system disorder, including schizophrenia and its related symptoms, such as dementia, psychosis, agitation, and irritability, especially without the side effects associated with dopamine antagonism and off-target muscarinic activation.

The following embodiments and aspects thereof are described and illustrated with compositions and methods, which are meant to be exemplary and illustrative, not limiting in scope. In various embodiments, one or more of the above-described problems have been reduced or eliminated, while other embodiments are directed to other improvements.

### SUMMARY

The present disclosure provides a method of treating a central nervous system disorder in a patient in need thereof, the method comprising administering a synergistic combination of a composition comprising xanomeline and/or a salt thereof and a composition comprising an antipsychotic.

The present disclosure further provides a method of treating schizophrenia in a patient in need thereof, the method comprising administering a synergistic combination of a composition comprising xanomeline and/or a salt thereof and a composition comprising risperidone or aripiprazole.

The present disclosure further provides a method of treating a central nervous system disorder in a patient in need thereof, the method comprising co-administering a first composition comprising xanomeline and/or a salt thereof and a composition comprising an antipsychotic, for example, wherein the first composition or the second composition or both are co-administered in a reduced amount compared to the first composition or the second composition used alone, such as in a subthreshold amount or in a lower dose. In certain embodiments, the first composition further comprises trospium chloride. In certain embodiments, the second composition comprises aripiprazole or risperidone.

Further aspects and advantages will be apparent to those of ordinary skill in the art from a review of the following detailed description. While the dosage form, method of making, and treatment method are susceptible of embodiments in various forms, the description hereafter includes specific embodiments to understand that the disclosure is illustrative and is not intended to limit the disclosure to the specific embodiments described herein.

### BRIEF DESCRIPTION OF THE DRAWINGS

The disclosure will be readily understood by the following detailed description in conjunction with the accompanying drawings, wherein like reference numerals designate like structural elements. The drawings provide exemplary embodiments or aspects of the disclosure and do not limit the scope of the disclosure.
FIG. 1 shows compiled data over independent studies with xanomeline, risperidone, and aripiprazole each analyzed separately via one-way repeated-measures ANOVA.
FIG. 2 shows the effects of subthreshold doses of risperidone and xanomeline on avoidance responses.
FIG. 3 shows the mean of differences between additive and actual responses in mice from 1 mg/kg xanomeline and 0.03 mg/kg risperidone.
FIG. 4 shows the mean of differences between additive and actual responses in mice from 1 mg/kg xanomeline and 0.1 mg/kg risperidone.
FIG. 5 shows the mean of differences between additive and actual responses in mice from 1 mg/kg xanomeline and 0.3 mg/kg risperidone.
FIG. 6 shows the effects of subthreshold doses of aripiprazole and xanomeline on avoidance responses.
FIG. 7 shows the mean of differences between additive and actual responses in mice from 1 mg/kg xanomeline and 1 mg/kg aripiprazole.
FIG. 8 shows the mean of differences between additive and actual responses in mice from 1 mg/kg xanomeline and 3 mg/kg aripiprazole.
FIG. 9 shows the mean of differences between additive and actual responses in mice from 3 mg/kg xanomeline and 1 mg/kg aripiprazole.
FIG. 10 shows the mean of differences between additive and actual responses in mice from 3 mg/kg xanomeline and 3 mg/kg aripiprazole.
FIG. 11 shows xanomeline's (Xan) efficacy is absent following blockade of central muscarinic receptors by scopolamine (SCOP), but not following blockade of a peripherally restricted muscarinic antagonist *N*-methylscopolamine (NMS; red and pink above).
FIG. 12 shows a dose of scopolamine (0.3 mg/kg) that fully reversed xanomeline efficacy, failed to attenuate risperidone's antipsychotic-like efficacy in conditioned avoidance response (CAR).
FIG. 13 shows a subthreshold dose of risperidone following MK-801 challenge. Mean (± SEM) total distance traveled risperidone dose-response curve.
FIG. 14 shows co-administration of xanomeline with a subthreshold dose of risperidone synergizes locomotor activity following the MK-801 challenge. Mean (± SEM) total distance traveled risperidone dose-response curve and synergy with xanomeline with sub-efficacious doses of risperidone.
FIG. 15 shows the effects of aripiprazole treatment on avoidance responding with or without xanomeline.
FIG. 16 shows the mean of differences between additive and actual responses in mice from 1 mg/kg xanomeline and 0.1 mg/kg aripiprazole.
FIG. 17 shows the mean of differences between additive and actual responses in mice from 1 mg/kg xanomeline and 0.3 mg/kg aripiprazole.
FIG. 18 shows the effect of scopolamine on aripiprazole activity in reducing avoidance responses.

### DETAILED DESCRIPTION

### Method of Treating

The present disclosure provides a method of treating a central nervous system disorder in a patient in need thereof. The method comprises administering a synergistic combination of a composition comprising xanomeline and/or a salt thereof and a composition comprising an antipsychotic. Also provided is a method comprising administering a synergistic combination of a first composition comprising xanomeline and/or a salt thereof and trospium chloride, and a second composition comprising an antipsychotic.

"Synergy" refers to the interaction or cooperation of two or more drugs that produce a combined effect in a subject that is greater than the sum of their separate effects in that subject. Synergy is more than merely additive, and it is greater than augmentation relative to any of the individual drugs. For example, if Drug A produces an effect of 2 units and Drug B produces an effect of 2 units, an augmented result is an effect of at least 3 units; an additive result is an effect equal to 4 units. A synergistic effect is greater than 4 units, the upper boundary of which is inherently unpredictable and only discernable through experimentation. An augmented result includes additive results and synergistic effects.

Xanomeline and atypical antipsychotics target different receptor families; therefore, there is the potential for greater therapeutic benefit with adjunctive treatment. The administration of xanomeline with the atypical antipsychotic risperidone was investigated in mouse models predictive of antipsychotic activity to determine the potential for greater therapeutic benefit with adjunctive treatment. In certain embodiments, the efficacy of xanomeline is augmented by the antipsychotic, such as risperidone and aripiprazole. In some instances, the combined does of xanomeline and the antipsychotic significantly augmented the effect sizes of that observed for each agent alone, where the combined effect was statistically significant and more than additive. The adjunctive treatment with xanomeline and an antipsychotic provides greater therapeutic benefit to the patient in need thereof. The term "antipsychotic" refers to a drug that diminishes psychosis, hallucinations, or delusions. Antipsychotics include, but are not limited to haloperidol, droperidol, chlorpromazine, fluphenazine, perphenazine, prochlorperazine, thioridazine, trifluoperazine, mesoridazine, periciazine, promazine, triflupromazine, levomepromazine, promethazine, pimozide, chlorprothixene, flupentixol, thiothixene, zuclopenthixol, clozapine, olanzapine, risperidone, quetiapine, ziprasidone, amisulpride, asenapine, paliperidone, zotepine, aripiprazole, bifeprunox, and tetrabenazine.

First-generation antipsychotics ("typical antipsychotics") are considered effective for managing the "positive" symptoms of schizophrenia, including hallucinations, hearing voices, aggression/hostility, disorganized speech, and psychomotor agitation. However, the development of moving disorders limits this class of drugs (e.g. haloperidol and chlorpromazine). Potent dopamine-blockade of the D2 receptor induces these movement disorders, including drug-induced parkinsonism, akathisia, dystonia, tardive dyskinesia, and other side effects, such as sedation, weight gain, and prolactin changes. Low-potency antipsychotics block many receptors other than the primary target (dopamine receptors), such as cholinergic or histaminergic receptors, resulting in a higher incidence of side effects, such as sedation, weight gain, and hypotension.

Second-generation antipsychotics, also known as atypical antipsychotics, generally have a lower risk of extrapyramidal side effects and tardive dyskinesia than first-generation antipsychotics. Typical and atypical antipsychotic drugs are more comparable in their clinical efficacy, except for clozapine, an atypical antipsychotic with unique efficacy in treatment-resistant schizophrenia. Antipsychotic drugs differ from one another in dosing, route of administration, pharmacokinetics, side effect profile, and cost, factors that influence how to select an antipsychotic drug for individual patients. Typical antipsychotic drugs have largely been replaced with atypical antipsychotics, such as risperidone, olanzapine, clozapine, quetiapine, aripiprazole, and ziprasidone.

Aripiprazole (Abilify^{™}) is an atypical antipsychotic orally indicated for treating schizophrenia, bipolar I, major depressive disorder, irritability associated with autism, and Tourette's syndrome. It is also indicated as an injection for agitation associated with schizophrenia or bipolar mania. Aripiprazole exerts its effects through partial agonism of dopamine and 5-HT1A receptors and antagonism of alpha-adrenergic and 5-HT2A receptors. The dosing for aripiprazole approved by the US Food and Drug Administration (FDA) is shown in Table 1. Oral formulations are administered once daily without regard to meals.

**Table 1 - Approved aripiprazole dosing**

| **Indication** | **Initial Dose** | **Recommended Dose** | **Maximum Dose** |
|---|---|---|---|
| Schizophrenia - adults | 10-15 mg/day | 10-15 mg/day | 30 mg/day |
| Schizophrenia - adolescents | 2 mg/day | 10 mg/day | 30 mg/day |
| Bipolar mania - adults: monotherapy | 15 mg/day | 15 mg/day | 30 mg/day |
| Bipolar mania - adults: adjunct to lithium or valproate | 10-15 mg/day | 15 mg/day | 30 mg/day |
| Bipolar mania - pediatric patients: monotherapy or adjunct to lithium or valproate | 2 mg/day | 10 mg/day | 30 mg/day |
| Major Depressive Disorder - adults adjunct to antidepressants | 2-5 mg/day | 5-10 mg/day | 15 mg/day |
| Irritability associated with autistic disorder - pediatric patients | 2 mg/day | 5-10 mg/day | 15 mg/day |
| Tourette's disorder - patients < 50 kg | 2 mg/day | 5 mg/day | 10 mg/day |
| Tourette's disorder - patients ≥ 50 kg | 2 mg/day | 10 mg/day | 20 mg/day |
| Agitation associated with schizophrenia or bipolar mania - adults | 9.75 mg/1.3 mL inject IM | - | 30 mg/day injected IM |

Chlorpromazine (Thoraxine^{™}) is a prototypical phenothiazine antipsychotic drug. The FDA-approved oral dosing for chlorpromazine for schizophrenia, psychoses, anxiety, and agitation is, to begin with, 25 mg three times daily or 75 mg at bedtime, increasing by daily amounts of 25 mg to an effective maintenance dose. The effective dose is usually between 75 and 300 mg daily, but some patients may require up to 1 g daily. Children under one year old should not use chlorpromazine unless it is lifesaving. Patients 1-5 years old should begin with 0.5 mg/kg bodyweight every 4-6 hours to a maximum recommended dose of 40 mg daily. Patients 6-12 years old should use a third to half of the adult dose to a maximum recommended dose of 75 mg daily. The elderly should start with a third to a half the usual adult dose with a more gradual increase in dosage.

Intramuscular dosing in adults for acute relief of symptoms should be 25-50 mg every 6-8 hours. Patients 1-5 years old should dose at 0.5 mg/kg bodyweight every 6-8 hours, not to exceed 40 mg daily. Patients 6-12 years old should do at 0.5 mg/kg bodyweight every 6-8 hours, not to exceed 75 mg daily. For the elderly, doses in the lower range for adults should be sufficient to control symptoms, for example, 25 mg every 8 hours.

Clozapine (Clozaril^{™}, Fazaclo^{™}, Versacloz^{™}) is a tricyclic dibenzodiazepine, classified as an atypical antipsychotic. The FDA-approved dosing for clozapine is 12.5 mg once or twice daily and then be continued with daily dosage increments of 25-50 mg/day, if well-tolerated, to achieve a target dose of 300-450 mg/day by the end of two weeks. Subsequent dosage increments should be made no more than once or twice weekly, in increments not to exceed 100 mg.

Flupentixol (Depixol^{™}, Fluanxol^{™}) is an antipsychotic drug of the thioxanthene group.

Flupentixol oral tablets are available at doses of 0.5 mg, 3 mg, and 5 mg. The dosage of flupentixol tablets should be individualized and adjusted according to the severity of symptoms and tolerance to the drug. The maintenance dose can usually be given as a single morning dose.

Fluphenazine is a phenothiazine used to treat psychoses. Fluphenazine Decanoate Injection, USP is available as a clear, pale yellow solution for intramuscular (IM) or subcutaneous (SC, SQ) use providing 25 mg fluphenazine decanoate per mL in a sesame oil vehicle with 12 mg benzyl alcohol as a preservative. For most patients, a dose of 12.5 to 25 mg (0.5 to 1 mL) may initiate therapy.

Haloperidol is a highly potent typical antipsychotic and one of the most frequently used antipsychotic medications worldwide. The initial dosage for oral haloperidol in adults with moderate symptoms is 0.5 mg to 2 mg b.i.d. or t.i.d. and with severe symptoms 3 mg to 5 mg b.i.d. or t.i.d. To achieve prompt control, higher doses may be required in some cases. The recommended doses for geriatric or debilitated patients is 0.5 mg to 2 mg b.i.d. or t.i.d. For chronic or resistant patients is 3 mg to 5 mg b.i.d. or t.i.d. Patients who remain severely disturbed or inadequately controlled may require dosage adjustment. Daily dosages up to 100 mg may be necessary in some cases for a response.

For children between the ages of 3 and 12 years (weight range 15 kg to 40 kg), oral haloperidol should begin at the lowest dose possible (0.5 mg per day). If required, the dose should be increased by 0.5 mg at 5 to 7-day intervals until the desired therapeutic effect is obtained. The total dose may be divided, to be given b.i.d. or t.i.d. The dosage for psychotic disorders is 0.05 mg/kg/day to 0.15 mg/kg/day. For nonpsychotic behavior disorders and Tourette's disorder, the recommended dosage is 0.05 mg/kg/day to 0.075 mg/kg/day.

Haloperidol is available as a sterile parenteral form for intramuscular injection. The injection provides 5 mg haloperidol (lactate) and lactic acid for pH adjustment between 3.0-3.6. For patients previously maintained on low doses of antipsychotics (e.g., up to the equivalent of 10 mg/day oral haloperidol), the initial dose of haloperidol decanoate is recommended to be 10-15 times the previous daily dose in oral haloperidol equivalents.

Olanzapine (Zyprexa^{™}) is a thienobenzodiazepine classified as an atypical antipsychotic. Recommended dosing is shown in Table 2.

**Table 2 - Recommended dosing of Olanzapine**

| **Indication** | **Dosing** |
|---|---|
| Schizophrenia in adults | Oral: Start at 5-10 mg once daily; Target: 10 mg/day within several days |
| Schizophrenia in adolescents | Oral: Start at 2.5-5 mg once daily; Target: 10 mg/day |
| Bipolar I Disorder (manic or mixed episodes) in adults | Oral: Start at 10 or 15 mg once daily |
| Bipolar I Disorder (manic or mixed episodes) in adolescents | Oral: Start at 2.5-5 mg once daily; Target: 10 mg/day |
| Bipolar I Disorder (manic or mixed episodes) with lithium or valproate in adults | Oral: Start at 10 mg once daily |
| Agitation associated with Schizophrenia and Bipolar I Mania in adults | IM: 10 mg (5 mg or 7.5 mg when clinically warranted) Assess for orthostatic hypotension before subsequent dosing (max. three doses 2-4 hours apart) |
| Depressive Episodes associated with Bipolar I Disorder in adults | Oral in combination with fluoxetine: Start at 5 mg of oral olanzapine and 20 mg of fluoxetine once daily |
| Depressive Episodes associated with Bipolar I Disorder in children and adolescents | Oral in combination with fluoxetine: Start at 2.5 mg of oral olanzapine and 20 mg of fluoxetine once daily |
| Treatment-Resistant Depression in adults | Oral in combination with fluoxetine: Start at 5 mg of oral olanzapine and 20 mg of fluoxetine once daily |

The lower starting dose is recommended in debilitated or pharmacodynamically sensitive patients, patients with a predisposition to hypotensive reactions, or with the potential for slowed metabolism.

Quetiapine (Seroquel^{™}) is an atypical antipsychotic used to treat schizophrenia, major depression, and bipolar disorder. Recommended doses are shown in Table 3.

**Table 3 - Recommended doses for quetiapine**

| **Indication** | **Initial Dose** | **Recommended Dose** | **Maximum Dose** |
|---|---|---|---|
| Schizophrenia-Adults | 25 mg twice daily | 150-750 mg/day | 750 mg/day |
| Schizophrenia-Adolescents (13-17 years) | 25 mg twice daily | 400-800 mg/day | 800 mg/day |
| Bipolar Mania-Adults Monotherapy or as an adjunct to lithium or divalproex | 50 mg twice daily | 400- 800 mg/day | 800 mg/day |
| Bipolar Mania-Children and Adolescents (10 to 17 years), Monotherapy | 25 mg twice daily | 400-600 mg/day | 600 mg/day |
| Bipolar Depression-Adults | 50 mg once daily at bedtime | 300 mg/day | 300 mg/day |

Risperidone (Perseris^{™}, Risperdal^{™}) is an atypical antipsychotic medication for treating several mood and mental health conditions, including schizophrenia and bipolar disorder. Another commonly used atypical antipsychotic is paliperidone, the primary active metabolite of risperidone *(i.e.,* 9-hydroxyrisperidone). Recommended dosing is shown in Table 4.

**Table 4 - Recommended doses for Risperidone**

| **Indication** | **Initial Dose** | **Titration** | **Target Dose** | **Effective Dose Range** |
|---|---|---|---|---|
| Schizophrenia - adults | 2 mg/day | 1-2 mg daily | 4-8 mg daily | 4-16 mg/day |
| Schizophrenia - adolescents | 0.5 mg/day | 0.5-1 mg daily | 3 mg/day | 1-6 mg/day |
| Bipolar mania - adults | 2-3 mg/day | 1 mg daily | 1-6 mg/day | 1-6 mg/day |
| Bipolar mania in children/ adolescents | 0.5 mg/day | 0.5-1 mg daily | 2.5 mg/day | 0.5-6 mg/day |
| Irritability associated with autistic disorder | 0.25 mg/day (<20 kg) 0.5 mg/day (≥20 kg) | 0.25-0.5 mg at ≥ 2 weeks | 0.5 mg/day (<20 kg) 1 mg/day (≥20 kg) | 0.5-3 mg/day |

Methods of treatment comprising a synergistic combination of two agents, such as a synergistic combination of xanomeline or a salt thereof and an antipsychotic, typically use lower dosages of one or both agents in the synergistic combination. In certain embodiments, the dose for the xanomeline or the antipsychotic or both is reduced compared to what the patient would receive for each agent alone. The doses of one agent or both agents can be reduced by such as by at least 10%, at least 20%, at least 30%, at least 40%, at least 50%, at least 60%, at least 70%, at least 80%, or at least 90%. These lower doses reduce toxicity and harmful side effects. Thus, synergistic combinations of xanomeline or a salt thereof and an antipsychotic provide superior safety and efficacy than monotherapies or combinations of therapies that do not act synergistically.

The combinations of xanomeline with an antipsychotic offer several advantages. The therapeutically effective dose of each can be lowered, thus avoiding side effects from each the xanomeline and the antipsychotic. In certain embodiments, the combination provides the advantage of achieving efficacy at a subthreshold dose. In certain embodiments, the combination provides the advantage of greater tolerability. In certain embodiments, the combination provides the advantage of fewer side effects.

In certain embodiments, the ratio of xanomeline to antipsychotic is between 3:1 and 1:3, such as 3:1, 2:1, 1:1, 1:2, or 1:3. In certain embodiments, the ratio of xanomeline to antipsychotic is 3:1. In certain embodiments, the ratio of xanomeline to antipsychotic is 1:1. In certain embodiments, the ratio of xanomeline to antipsychotic is 1:3. In certain embodiments, the ratio of xanomeline to antipsychotic is between 1:0.03 and 1:0.3, such as between 1:0.03 and 1:0.05, between 1:0.05 and 1:0.1, between 1:0.1 and 1:0.2, or between 1:0.2 and 1:0.3. In certain embodiments, the ratio of xanomeline to antipsychotic is 1:0.03. In certain embodiments, the ratio of xanomeline to antipsychotic is 1:0.1. In certain embodiments, the ratio of xanomeline to antipsychotic is 1:0.3.

In certain embodiments, the ratio of xanomeline to aripiprazole is between 3:1 and 1:3, such as 3:1, 2:1, 1:1, 1:2, or 1:3. In certain embodiments, the ratio of xanomeline to aripiprazole is 3:1. In certain embodiments, the ratio of xanomeline to aripiprazole is 1:1. In certain embodiments, the ratio of xanomeline to aripiprazole is 1:3. In certain embodiments, the ratio of xanomeline to aripiprazole is between 1:0.03 and 1:0.3, such as between 1:0.03 and 1:0.05, between 1:0.05 and 1:0.1, between 1:0.1 and 1:0.2, or between 1:0.2 and 1:0.3. In certain embodiments, the ratio of xanomeline to aripiprazole is 1:0.03. In certain embodiments, the ratio of xanomeline to aripiprazole is 1:0.1. In certain embodiments, the ratio of xanomeline to aripiprazole is 1:0.3.

In certain embodiments, the ratio of xanomeline to risperidone is 1:3. In certain embodiments, the ratio of xanomeline to risperidone is between 1:0.03 and 1:0.3, such as between 1:0.03 and 1:0.05, between 1:0.05 and 1:0.1, between 1:0.1 and 1:0.2, or between 1:0.2 and 1:0.3. In certain embodiments, the ratio of xanomeline to risperidone is 1:0.03. In certain embodiments, the ratio of xanomeline to risperidone is 1:0.1. In certain embodiments, the ratio of xanomeline to risperidone is 1:0.3.

In some studies, the xanomeline and antipsychotic were co-administered on a per weight basis of mg/kg/day. The average body mass, globally, is 136 pounds (62 kg). In North America, which has the highest average body mass of any continent, the number is 178 pounds (80.7 kg). Based on these numbers, one of skill in the art can convert the daily mg/kg dosing to total daily doses in mg and select available drug products for dosing. An example is shown in Table 5, based on the method from Reagan-Shaw et al., The FASEB Journal, 22(3), 659-661.

**Table 5 - Daily dose conversions**

| **Dosage in mice** | **Converted to dosage in humans** | **For 60-kg patient** | **For 80-kg patient** |
|---|---|---|---|
| 0.03 mg/kg | 0.0024 mg/kg | 0.14 mg | 0.19 mg |
| 0.10 mg/kg | 0.0081 mg/kg | 0.49 mg | 0.64 mg |
| 0.30 mg/kg | 0.024 mg/kg | 1.5 mg | 1.9 mg |
| 1.0 mg/kg | 0.081 mg/kg | 4.9 mg | 6.5 mg |
| 3.0 mg/kg | 0.24 mg/kg | 15mg | 19 mg |

Generally, xanomeline was tested at 1 mg/kg/day in mice, which corresponds to 0.081 mg/kg for humans (4.9-6.5 mg per day) or 3 mg/kg/day in mice which (15-19 mg per day). The amounts of xanomeline tested in the clinic without an antipsychotic are between 25 and 225 mg per day, what are far higher than in the present examples. Risperidone was tested at 0.03-0.3 mg/kg/day (0.14-1.9 mg). The effective dose range for monotherapy is 4-16 mg per day. Aripiprazole was tested over a wider range, including dosing of 0.1-0.3 mg/kg/day (0.14-1.9 mg). The recommended daily dose of aripiprazole monotherapy is 10-15 mg and the maximum daily dose of 30 mg.

In some embodiments, a synergistic amount of the composition comprising xanomeline and/or a salt thereof and the composition comprising an antipsychotic is a specific concentration of the composition comprising xanomeline and/or a salt thereof and the composition comprising the antipsychotic. In some embodiments, a synergistic amount of the composition comprising xanomeline and/or a salt thereof and the composition comprising the antipsychotic is a specific ratio of the composition comprising xanomeline and/or a salt thereof to the composition comprising the antipsychotic.

As used herein, "co-administer" and "co-administration" and variants thereof mean the administration of at least two drugs to a patient either subsequently, simultaneously, or consequently proximate in time to one another (e.g., within the same day, or week or period of 30 days, or sufficiently proximate that each of the at least two drugs can be simultaneously detected in the blood plasma). When co-administered, two or more active agents can be co-formulated as part of the same composition or administered as separate formulations. This also may be referred to herein as "concomitant" administration or variants thereof.

The synergistic effect of the composition comprising xanomeline and/or a salt thereof and the composition comprising an antipsychotic may include, but is not limited to an effect of treating a central nervous system disorder, e.g., by reduced dementia, psychosis, agitation, irritability, a change from Baseline in Positive and Negative Syndrome Scale (PANSS) Total Score or Positive Score or Negative Score, the number of participants with each Clinical Global Impression - Severity (CGI-S) Score at baseline, change from baseline in the PANSS Marder Factor Score, and the percentage of participants who were CGI-S responders.

In certain embodiments, the composition comprising xanomeline and/or a salt thereof further comprises an anticholinergic agent. In certain embodiments, the anticholinergic agent is a muscarinic antagonist. In certain embodiments, the muscarinic antagonist is chosen from trospium, tolterodine, darifenacin, solifenacin, fesoterodine, scopolamine, N-methylscopolamine, and a salt thereof. In certain embodiments, the muscarinic agonist is trospium chloride.

In one embodiment, xanomeline combined with trospium chloride and an antipsychotic treats an animal. In a further embodiment, the animal is a mammal. In an embodiment, the mammal is a human being.

In one embodiment, trospium chloride decreases the side effects associated with xanomeline. Such side effects include, but are not limited to, GI side effects, cardiac side effects, excessive sweating, and excessive salivation. The use of trospium with xanomeline allows the xanomeline to be used clinically when the xanomeline would not otherwise be used clinically due to its side effects. In another embodiment, the use of trospium chloride with the xanomeline allows for the xanomeline to achieve a higher maximum tolerated dose than xanomeline would otherwise achieve, with or without co-administration with a composition comprising an antipsychotic.

Various time and resource-intensive methods may be used to demonstrate the efficacy of the combinations of xanomeline and an antipsychotic and, optionally, trospium chloride. For example, animal models demonstrate the efficacy of new therapeutics for schizophrenia, including pharmacological models (e.g., ketamine model) and genetic models (e.g., DISCI mouse). Likewise, animal models, including rodents, dogs, and non-human primates, demonstrate the side effect profile of pharmacological agents. Animal models are an experimental proxy for humans but may suffer from deficiencies in the physiological differences between humans and animals and may have limited predictive power for human experiments, particularly for central nervous system disorders.

Alternatively, the disclosed combinations can be tried in controlled clinical trials of people. Standard measures based on patient self-report can be used by those skilled in the art to assess various side effects such as GI discomfort. As another example, objective physiological measures (e.g., EKGs) may be used by those skilled in the art. A set of standard measures has also been developed to assess schizophrenia symptoms, including the Brief Psychiatric Rating Scale (BPRS), the Positive and Negative Syndrome Scale (PANSS), and Clinical Global Impression (CGI). Typically, clinical trials are double-blinded, where one group of patients receives an inactive placebo, and the other group the active intervention.

The Positive and Negative Syndrome Scale (PANSS) is a medical scale used for measuring symptom severity of patients with schizophrenia. The name refers to the two types of symptoms in schizophrenia, as defined by the American Psychiatric Association: positive symptoms, which refer to an excess or distortion of normal functions (e.g., hallucinations and delusions), and negative symptoms, which represent a diminution or loss of normal functions. Some of these functions which may be lost include normal thoughts, actions, the ability to tell fantasies from reality, and the ability to properly express emotions

The PANSS is a relatively brief interview of about 45 to 50 minutes. The interviewer must be trained to a standardized level of reliability. The patient is rated from 1 to 7 on 30 different symptoms in three categories based on the interview and reports of family members or primary care hospital workers. The first category of the PANSS is the positive scale, comprising 7 Items (minimum score = 7, maximum score = 49): delusions, conceptual disorganization, hallucinations, excitement, grandiosity, suspiciousness/persecution, and hostility. The second category is the negative scale, comprising 7 items (minimum score = 7, maximum score = 49): blunted affect, emotional withdrawal, poor rapport, passive/apathetic social withdrawal, difficulty in abstract thinking, lack of spontaneity, and flow of conversation, stereotyped thinking. The third category is the General Psychopathology scale, which comprises 16 items (minimum score = 16, maximum score = 112): somatic concern, anxiety, guilt feelings, tension, mannerisms and posturing, depression, motor retardation, uncooperativeness, unusual thought content, disorientation, poor attention, lack of judgment and insight, disturbance of volition, poor impulse control, preoccupation, active social avoidance.

PANSS Marder factor score is the sum of five negative scales and two general scales (N1. Blunted affect; N2. Emotional withdrawal; N3. Poor rapport; N4. Passive/apathetic social withdrawal; N6. Lack of spontaneity; G7. Motor retardation; and G16. Active social avoidance). If a patient has a PANSS assessment recorded, but any of the items are missing, the last non-missing score for the individual item from previous assessments will be carried forward. If more than 30% of the items are missing at a particular visit, the respective positive score is not calculated. It is treated as missing data in the analysis.

Because 1 rather than 0 is given the lowest score for each item, a patient cannot score lower than 30 for the total PANSS score. Subscores can be given separately for the positive items, negative items, and general psychopathology. The maximum possible total score is 210. In the original publication on the PANSS scale, 101 adult patients (20-68 years old) with schizophrenia were ranked. Their mean scores were a positive scale of 18.20, a negative scale of 21.01, and general psychopathology of 37.74. The mean total PANSS score for these subjects was 76.95.

In certain embodiments, the Positive and Negative Syndrome Scale (PANSS) total score for the subject decreases by at least 10 points than the placebo, for example, after five treatment weeks. In certain embodiments, the PANSS positive subscore decreases by at least 3 points than the placebo, for example, after five treatment weeks. In certain embodiments, the PANSS negative subscore decreases by at least 2 points than the placebo, for example, after five treatment weeks.

Another scale used to assess patients is the Clinical Global Impression - Severity scale (CGI-S). This 7-point scale requires the clinician to rate the severity of the patient's illness at the time of assessment relative to the clinician's experience with patients who have the same diagnosis. Possible ratings are (1) Normal, not at all ill; (2) Borderline mentally ill; (3) Mildly ill, (4) Moderately ill; (5) Markedly ill; (6) Severely ill, and (7) Among the most extremely ill patients. Among schizophrenia patients, changes in the CGI-S follow a consistent pattern relative to more objective PANSS scoring.

Before co-administering the disclosed combinations, patients may have a lead-in period from one to 28 days, during which lead-in period trospium chloride and/or the antipsychotic is given alone. In one embodiment, the trospium chloride and/or antipsychotic are co-administered for one or more dose periods before co-administering xanomeline to accumulate trospium chloride and/or antipsychotic in the body or for the trospium chloride and/or antipsychotic to reach or approach steady-state exposure levels. This accumulation or higher exposure levels of the trospium chloride increases the blockade of muscarinic receptors outside of the brain and reduces adverse events when xanomeline is administered. Likewise, this accumulation or higher exposure levels to the antipsychotics increase the blockade of D2 receptors and simplifies the process for titrating to the synergistic amount of xanomeline and/or a salt thereof. In another embodiment, the trospium chloride and/or antipsychotic are co-administered for one or more days before xanomeline.

In certain embodiments, the patient was already taking a steady-state dose of an antipsychotic before beginning treatment with KarXT. In this situation, after an initial treatment, the dose of the antipsychotic may be titrated down to account for its synergistic effect with KarXT. Likewise, the dose of KarXT may be titrated down to account for its synergistic effect with the antipsychotic.

In certain embodiments, the total daily dose is 5 mg a salt of trospium.

In certain embodiments, the total daily dose is 10 mg a salt of trospium.

In certain embodiments, the total daily dose is 15 mg a salt of trospium.

In certain embodiments, the total daily dose is 17.5 mg a salt of trospium.

In certain embodiments, the total daily dose is 20 mg a salt of trospium.

In certain embodiments, the total daily dose is 30 mg a salt of trospium.

In certain embodiments, the total daily dose is 25 mg xanomeline and/or the salt thereof.

In certain embodiments, the total daily dose is 50 mg xanomeline and/or the salt thereof.

In certain embodiments, the total daily dose is 75 mg xanomeline and/or the salt thereof.

In certain embodiments, the total daily dose is 100 mg xanomeline and/or the salt thereof.

In certain embodiments, the total daily dose is 150 mg xanomeline and/or the salt thereof.

In certain embodiments, the total daily dose is 175 mg xanomeline and/or the salt thereof.

In certain embodiments, the total daily dose is 200 mg xanomeline and/or the salt thereof.

In certain embodiments, the total daily dose is 25 mg xanomeline and/or the salt thereof and 5 mg a salt of trospium.

In certain embodiments, the total daily dose is 50 mg xanomeline and/or the salt thereof and 10 mg a salt of trospium.

In certain embodiments, the total daily dose is 50 mg xanomeline and/or the salt thereof and 20 mg a salt of trospium.

In certain embodiments, the total daily dose is 75 mg xanomeline and/or the salt thereof and 15 mg a salt of trospium.

In certain embodiments, the total daily dose is 75 mg xanomeline and/or the salt thereof and 30 mg a salt of trospium.

In certain embodiments, the total daily dose is 100 mg xanomeline and/or the salt thereof and 17.5 mg a salt of trospium.

In certain embodiments, the total daily dose is 100 mg xanomeline and/or the salt thereof and 20 mg a salt of trospium.

In certain embodiments, the total daily dose is 100 mg xanomeline and/or the salt thereof and 40 mg a salt of trospium.

In certain embodiments, the total daily dose is 150 mg xanomeline and/or the salt thereof and 20 mg a salt of trospium.

In certain embodiments, the total daily dose is 150 mg xanomeline and/or the salt thereof and 30 mg a salt of trospium.

In certain embodiments, the total daily dose is 150 mg xanomeline and/or the salt thereof and 40 mg a salt of trospium.

In certain embodiments, the total daily dose is 175 mg xanomeline and/or the salt thereof and 30 mg a salt of trospium.

In certain embodiments, the total daily dose is 175 mg xanomeline and/or the salt thereof and 40 mg a salt of trospium.

In certain embodiments, the total daily dose is 200 mg xanomeline and/or the salt thereof and 30 mg a salt of trospium.

In certain embodiments, the total daily dose is 200 mg xanomeline and/or the salt thereof and 40 mg a salt of trospium.

In one embodiment, xanomeline and trospium chloride are administered to a patient 6 times during a 24-hour period. In another embodiment, xanomeline and trospium chloride are administered to a patient 5 times during a 24-hour period. In another embodiment, xanomeline and trospium chloride are administered to a patient 4 times during a 24-hour period. In an embodiment, xanomeline and trospium chloride are administered to a patient 3 times during a 24-hour period. In another embodiment, xanomeline and trospium chloride are administered to a patient twice during a 24-hour period. In another embodiment, xanomeline and trospium chloride are administered to a patient once during a 24-hour period.

In certain embodiments, the administration comprises a dosing schedule of 25 mg xanomeline and/or the salt thereof and 5 mg a salt of trospium twice daily.

In certain embodiments, the administration comprises a dosing schedule of 25 mg xanomeline and/or the salt thereof and 5 mg a salt of trospium thrice daily.

In certain embodiments, the administration comprises a dosing schedule of 25 mg xanomeline and/or the salt thereof and 5 mg a salt of trospium twice daily, and 50 mg xanomeline and/or the salt thereof and 7.5 mg a salt of trospium once daily.

In certain embodiments, the administration comprises a dosing schedule of 25 mg xanomeline and/or the salt thereof and 10 mg a salt of trospium twice daily.

In certain embodiments, the administration comprises a dosing schedule of 50 mg xanomeline and/or the salt thereof and 7.5 mg a salt of trospium twice daily, and 50 mg xanomeline and/or the salt thereof and 5 mg a salt of trospium once daily.

In certain embodiments, the administration comprises a dosing schedule of 50 mg xanomeline and/or the salt thereof and 10 mg a salt of trospium twice daily.

In certain embodiments, the administration comprises a dosing schedule of 50 mg xanomeline and/or the salt thereof and 10 mg a salt of trospium thrice daily.

In certain embodiments, the administration comprises a dosing schedule of 50 mg xanomeline and/or the salt thereof and 10 mg a salt of trospium twice daily, and 75 mg xanomeline and/or the salt thereof and 10 mg a salt of trospium once daily.

In certain embodiments, the administration comprises a dosing schedule of 75 mg xanomeline and/or the salt thereof and 10 mg a salt of trospium twice daily, and 50 mg xanomeline and/or the salt thereof and 10 mg a salt of trospium once daily.

In certain embodiments, the low doses are split TID rather than BID. BID keeps the trospium dose as low as possible with a minimum amount of 10 mg of trospium per capsule. In certain embodiments, a starting dose of 50/5 mg or 50/7.5 mg xanomeline/trospium is split TID so the individual dose 17/2.5 mg xanomeline/trospium.

Treatment may be initiated with smaller dosages. After that, small increments may increase the dosage until a balance between therapeutic effect and side effects is attained. While the subject is being treated, the patient's health may be monitored by measuring one or more of the relevant indices at predetermined times during the treatment period. Treatment, including composition, amounts, administration, and formulation times, may be adjusted per such monitoring. The patient may be periodically reevaluated to determine improvement by measuring the same parameters. Adjustments to the disclosed composition administered and possibly to the administration time may be made based on these reevaluations.

In certain embodiments, the patient has a diagnosis of schizophrenia. In certain embodiments, the patient has acute psychosis. In certain embodiments, the patient has psychosis associated with Alzheimer's disease. In certain embodiments, the patient has a schizo-affective disorder. In certain embodiments, the patient has psychosis. In certain embodiments, the patient has a delusional disorder. In certain embodiments, the patient has psychosis associated with Parkinson's disease. In certain embodiments, the patient has psychotic depression. In certain embodiments, the patient has bipolar disorder. In certain embodiments, the patient has bipolar disorder with psychosis. In certain embodiments, the patient has Huntington's disease. In certain embodiments, the patient has Lewy Body dementia. In certain embodiments, the patient has dementia-related psychosis (DRP).

In certain embodiments, the patient previously had been administered one or more antipsychotics. In certain embodiments, the patient was an inadequate responder to such administration. In certain embodiments, the patient was treatment-resistant.

### Pharmaceutical Compositions

Provided herein is an oral pharmaceutical composition, comprising a plurality of xanomeline beads comprising xanomeline or a salt thereof; and a plurality of trospium beads comprising a salt of trospium. In certain embodiments, the salt of trospium is chosen from trospium chloride, trospium bromide, trospium iodide, and trospium saccharinate.

In some embodiments of the kits of the disclosure, the therapeutically effective amount of the composition comprising xanomeline and/or a salt thereof comprises a synergistically effective amount of the composition comprising xanomeline and/or a salt thereof. In some embodiments, the composition comprising xanomeline and/or a salt thereof and the composition comprising an antipsychotic exhibit synergy. Synergy between the composition comprising xanomeline and/or a salt thereof and the composition comprising an antipsychotic can be measured using the methods described herein.

In certain embodiments, the oral pharmaceutical composition comprises a plurality of xanomeline beads comprising xanomeline or a salt thereof. In certain embodiments, the oral pharmaceutical composition comprises a plurality of trospium beads comprising a salt of trospium.

In certain embodiments, the plurality of xanomeline beads has a core comprising xanomeline or a salt thereof. In certain embodiments, the plurality of trospium beads has a core comprising a salt of trospium.

In certain embodiments, a capsule shell comprising hydroxypropyl methylcellulose (HPMC) containing separate populations of drug beads containing xanomeline tartrate or trospium chloride wherein the drug beads are of comparable size and release the actives rapidly and at substantially similar rates. Following the dissolution of the capsule shell in the stomach, the drug beads may dissolve in the stomach or pass through the pyloric valve into the duodenum intact or partially intact. Still, the two drugs' ratio, both in dissolved form and in undissolved form, remains relatively constant in the gastrointestinal tract until the drugs are absorbed.

The formulation for each drug bead allows substantially similar performance from two actives at different dose ranges. The actives are released into the blood serum at substantially similar rates or achieve a substantially similar Tₘₐₓ. In certain embodiments, a capsule comprises 50 mg xanomeline as the tartrate salt and 10 mg trospium chloride. Fifty mg xanomeline as a free base corresponds to about 76 mg xanomeline tartrate.

A discrepancy in the number of drug beads in the capsule increases the probability that the drug beads' ratio would not remain substantially constant after the beads are released and disperse. Thus, in certain embodiments, the trospium beads are formulated with a lower drug load. Effective doses of trospium and xanomeline are contained in roughly equivalent numbers of beads. Despite the differences in drug loads in certain embodiments, the trospium and xanomeline beads release at roughly similar rates. For example, if the dissolution of the capsules is assessed using a United States Pharmacopeia (USP) dissolution apparatus, the percentage of xanomeline dissolved is substantially equivalent to the percentage of dissolved trospium chloride, such as at 10 min, 20 min, or 30 min.

The medicament may also include one or more pharmaceutically acceptable salts. The medicament may include one or more pharmaceutically-acceptable carriers. The medicament may be administered orally. The medicament may be delivered orally using tablets, troches, liquids, emulsions, suspensions, drops, capsules, caplets or gel caps, and other methods of oral administration known to one skilled in the art.

The medicament may be in a dosage form that immediately releases the drug. In an alternative embodiment, the medicament may have a controlled release dosage form.

The medicament may be in dosage forms that use other controlled-release formulations known to one in the art.

In another embodiment, the medicament is combined with one or more therapies, including psychotherapy and drugs. Therapeutic agents include, but are not limited, to antipsychotics, anxiolytics, anti-depressants, sedatives, tranquilizers, analgesics, and other pharmacological interventions known to one skilled in the art. A therapeutic agent may fall under the category of more than one drug. For instance, benzodiazepines can be considered anxiolytics, sedatives, and tranquilizers.

### Definitions

The articles "a" and "an" refer to one or more than one (i.e., to at least one) of the grammatical object of the article. By way of example, "an element" means one element or more than one element.

The terms "comprise" and "comprising" are inclusive, open sense, meaning that additional elements may be included.

The term "consisting" limits the elements to those specified except for impurities ordinarily associated in addition to that.

The term "consisting essentially of' limits those specified elements and those that do not materially affect the basic and novel characteristics of the material or steps.

All ranges set forth herein include all possible subsets of ranges and any combinations of such subset ranges. By default, ranges include the stated endpoints, unless stated otherwise, where a range of values is provided, each intervening value between the upper and lower limit of that range and any other stated or intervening value in that stated range is encompassed within the disclosure. The upper and lower limits of these smaller ranges may independently be included in the smaller ranges and encompassed within the disclosure, subject to any specifically excluded limit in the stated range. Where the stated range includes one or both limits, ranges excluding either or both of those included limits are also contemplated to be part of the disclosure.

The term "wt.%" is the weight percent based on the total weight, e.g., of the core, or enteric coating, or total bead, as described in context. Unless stated otherwise, the wt.% is intended to describe the weight percent based on dry weight (e.g., for a core following drying).

The term "controlled release" is defined as a prolonged-release pattern of one or more drugs, such that the drugs are released over a period. A controlled release formulation has release kinetics that results in measurable serum levels of the drug over a period longer than what would be possible following intravenous injection or following administration of an immediate release oral dosage form. Controlled release, slow-release, sustained-release, extended-release, prolonged-release, and delayed-release have the same definitions.

The term "including" means "including but not limited to." "Including" and "including but not limited to" are used interchangeably.

The term "mammal" is known in the art. Exemplary mammals include humans, primates, bovines, porcines, canines, felines, and rodents (e.g., mice and rats).

A "patient," "subject," or "host" to be treated by the subject method means either a human or non-human mammal.

"Elderly" or "geriatric" refer to a person aged 65 years or older.

The term "pharmaceutically-acceptable carrier" is art-recognized. It refers to a pharmaceutically-acceptable material, composition or vehicle, such as a liquid or solid filler, diluent, excipient, solvent, or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials that may serve as pharmaceutically acceptable carriers include sugars, such as lactose, glucose, and sucrose; starches, such as corn starch and potato starch; cellulose and its derivatives, such as sodium carboxymethyl cellulose, ethylcellulose, and cellulose acetate; powdered tragacanth; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

The term "pharmaceutically-acceptable salt" or "salt" is art-recognized. It refers to a salt prepared from relatively nontoxic acids or bases, including inorganic acids and bases and organic acids and bases, including, for example, those contained in compositions of the present disclosure. Suitable non-toxic acids include inorganic and organic acids such as acetic, benzenesulfonic, benzoic, camphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, saccharinate, succinic, sulfuric, tartaric acid, p-toluenesulfonic, hydrochloric, hydrobromic, phosphoric, and sulfuric acids and the like.

The term "treating" is art-recognized and refers to curing as well as ameliorating at least one symptom of any condition or disorder.

In jurisdictions that forbid the patenting of methods practiced on the human body, the meaning of "administering" of a composition to a human subject shall be restricted to prescribing a controlled substance that a human subject will self-administer by any technique (e.g., orally, inhalation, topical application, injection, insertion, etc.). The broadest reasonable interpretation consistent with laws or regulations defining patentable subject matter is intended. In jurisdictions that do not forbid the patenting of methods practiced on the human body, the "administering" of compositions includes both methods practiced on the human body and the foregoing activities.

The term "therapeutic agent" is art-recognized and refers to any chemical moiety that is a biologically, physiologically, or pharmacologically active substance acting locally or systemically in a subject. Examples of therapeutic agents, also referred to as "drugs," are described in well-known literature references such as *the* Merck Index (14th edition), the Physicians'Desk Reference (64th edition), and The Pharmacological Basis of Therapeutics (12th edition). These therapeutic agents include without limitation medicaments; vitamins; mineral supplements; substances used for the treatment, prevention, diagnosis, cure, or mitigation of a disease or illness; substances that affect the structure or function of the body, or pro-drugs, which become biologically active or more active after they have been placed in a physiological environment.

The term "psychotherapy" refers to non-pharmacological therapies. Those skilled in the art use various techniques involving verbal and other interactions with a patient to affect a positive therapeutic outcome. Such techniques include, but are not limited to, behavior therapy, cognitive therapy, psychodynamic therapy, psychoanalytic therapy, group therapy, family counseling, art therapy, music therapy, vocational therapy, humanistic therapy, existential therapy, transpersonal therapy, client-centered therapy (also called person-centered therapy), Gestalt therapy, biofeedback therapy, rational emotive behavioral therapy, reality therapy, response-based therapy, Sandplay therapy, status dynamics therapy, hypnosis, and validation therapy. Psychotherapy may involve combining two or more techniques. A therapist can select and adjust the techniques based on the individual patient's needs and responses.

The term "muscarinic disorder" refers to any disease or condition ameliorated by activating the muscarinic system. Such diseases include ones in which direct activation of muscarinic receptors themselves or inhibition of cholinesterase enzymes has produced a therapeutic effect.

The terms "diseases related to schizophrenia" and "disorders related to schizophrenia" include, but are not limited to, schizo-affective disorder, psychosis, including acute psychosis, delusional disorders, psychosis associated with Alzheimer's disease, psychosis associated with Parkinson's disease, psychotic depression, bipolar disorder, bipolar with psychosis, Huntington's disease, Lewy Body dementia, or any other disease with psychotic features.

"Psychosis" refers to an abnormal condition of the mind that results in difficulties determining what is real and not. Symptoms of psychosis include, but are not limited to, false beliefs (delusions), seeing or hearing things that others do not see or hear (hallucinations), incoherent speech, behavior that is inappropriate for the situation, sleep problems, social withdrawal, lack of motivation, and difficulties carrying out daily activities.

"Acute psychosis" refers to the quick or strong onset of psychotic symptoms in a patient, for example, as defined in "Acute and Transient Psychotic Disorder" (International Classification of Diseases-10) and "Brief Psychosis"(DSM-IV). A sharp striking delusion with quick changes in the structure occurs in the individual who has acute psychosis after a short preliminary period of anxiety, insomnia, and confusion. Acute psychosis can include acute psychotic exacerbation when a patient may respond to hallucinations or delusions. Acute psychosis lasts for a short time, typically from one to two weeks.

The term "activator" means a molecule described as an agonist, partial agonist, co-agonist, physiological agonist, potentiator, stimulator, allosteric potentiator, positive allosteric modulator, allosteric agonist, or a molecule that increases the activity or signaling of receptors directly or indirectly.

The term "inhibitor" means a molecule described as an antagonist, partial antagonist, competitive antagonist, non-competitive antagonist, uncompetitive antagonist, silent antagonist, inverse agonist, reversible antagonist, physiological antagonist, irreversible antagonist, inhibitor, reversible inhibitor, irreversible inhibitor, negative allosteric modulator, allosteric antagonist, or a molecule that decreases the activity or signaling of receptors directly or indirectly.

As used herein, an "adverse event" is any untoward medical occurrence associated with treatment with a pharmaceutical composition described herein. A "mild adverse event" is easily tolerated by the subject, causes minimal discomfort, and does not interfere with everyday activities. A "moderate adverse event" is sufficiently discomforting to interfere with everyday activities; intervention may be needed. A "severe adverse event" prevents everyday activities; treatment or other intervention is usually needed. A "serious adverse event" results in death; is life-threatening (immediate risk of death from the event as it occurred); requires or prolongs inpatient hospitalization; results in persistent or significant disability/incapacity; or results in a congenital anomaly/disability, cancer, or drug overdose. An adverse event is incapacitating or disabling if it results in a substantial or permanent disruption of the subject's ability to carry out normal life functions.

As used herein, a patient is said to "tolerate" a dose of a compound if administering that dose to that patient does not result in an unacceptable adverse event or an unacceptable combination of adverse events. One of skill in the art will appreciate that tolerance is a subjective measure and that what may be tolerable to one patient may not be tolerable to a different patient. For example, one patient may not be able to tolerate a headache. In contrast, a second patient may find headaches tolerable but is not able to tolerate vomiting. For a third patient, either headache alone or vomiting alone is tolerable. Still, the patient cannot tolerate the combination of headache and vomiting, even if the severity of each is less than when experienced alone.

The term "maximum tolerated dose" means the highest dose of a drug or therapeutic that a patient can take without the patient experiencing intolerable side effects. The maximum tolerated dose is typically determined empirically in clinical trials.

The term "muscarinic receptors" refers to G-protein linked receptors that bind the neurotransmitter acetylcholine. To date, five subtypes of the muscarinic receptor have been identified. "M1" means the subtype one muscarinic receptor. "M2" means the subtype two muscarinic receptor. "M3" means the subtype three muscarinic receptor. "M4" means the subtype four muscarinic receptor. "M5" means the subtype five muscarinic receptor.

The term "anxiolytics" refers to drugs that reduce anxiety, fear, panic, or related feelings. Such drugs include, but are not limited to, benzodiazepines (e.g., alprazolam, chlordiazepoxide, clonazepam, clorazepate, diazepam, lorazepam), buspirone, barbiturates (e.g., amobarbital, pentobarbital, secobarbital, phenobarbital), and hydroxyzine.

The term "anti-depressants" refers to drugs that alleviate depression and related conditions (e.g., dysthymia). Such drugs include, but are not limited to, selective serotonin-reuptake inhibitors (SSRIs, e.g., citalopram, escitalopram, fluoxetine, fluvoxamine, paroxetine, sertraline), serotonin-norepinephrine reuptake inhibitors (SNRIs, e.g., desvenlafaxine, duloxetine, milnacipran, venlafaxine), mianserin, mirtazapine, norepinephrine reuptake inhibitors (e.g., atomoxetine, mazindol, reboxetine, viloxazine), bupropion, tianeptine, agomelatine, tricyclic antidepressants (e.g., amitriptyline, clomipramine, doxepin, imipramine, trimipramine, desipramine, nortriptyline, protriptyline), and monoamine oxidase inhibitors (e.g., isocarboxazid, moclobemide, phenelzine, selegiline, tranylcypromine).

The terms "sedatives" or "tranquilizers" refer to drugs that induce somnolence, promote a feeling of being tired or desire to sleep, or promote a state of unconsciousness. Such drugs include, but are not limited to, benzodiazepines, barbiturates (e.g., amobarbital, pentobarbital, secobarbital, phenobarbital), eszopiclone, zaleplon, zolpidem, and zopiclone.

### EXAMPLES

The following examples are provided for illustration and are not intended to limit the scope of the disclosure.

### Example 1 - Rodent models of psychosis demonstrated synergy between xanomeline dosed with risperidone or aripiprazole.

Adult male C57BL/6J mice were tested for drug effects using conditioned avoidance response (CAR) and MK-801-induced locomotor activity (LMA) tests. In the CAR assay, mice were first trained to avoid a foot shock (i.e., crossing from one side of a shuttle box to another within 10 secs) to a performance criterion of >85% avoidance responses. A repeated-measures, counterbalanced design was used. Two experiments evaluated the effects of blocking central (with scopolamine) vs. peripheral (with *N*-methylscopolamine) muscarinic receptors. Additional experiments evaluated augmentation and synergy by combining dosing subthreshold doses of xanomeline plus risperidone or aripiprazole. "Subthreshold" of "sub-efficacious" refers to doses that are lower than the therapeutically effective amount for monotherapy but which could be effective in synergy with the antipsychotics tested.

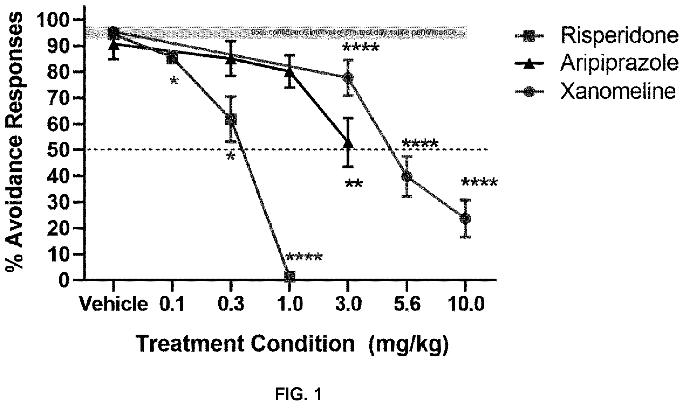

Xanomeline, risperidone, and aripiprazole dose-dependently reduced conditioned avoidance responding. Referring to FIG. 1, the compiled data over independent studies with each compound were analyzed separately via one-way repeated-measures ANOVA. A Dunnett post-hoc test was performed and that dose group significance was only assessed against Vehicle. Xanomeline [F(2.8, 55.4) = 30.62, p<0.0001], N = 21; Risperidone [F(1.17, 9.34) = 83.96, p < 0.0001]. N = 9; Aripiprazole [F(2.16, 21.65) = 7.12, p = 0.0036], N = 11. Values are mean ± SEM percent avoidance responses on test day. ^{∗}p<0.05, ^{∗∗} p<0.01, ^{∗∗∗∗} p<0.0001 vs. Vehicle.

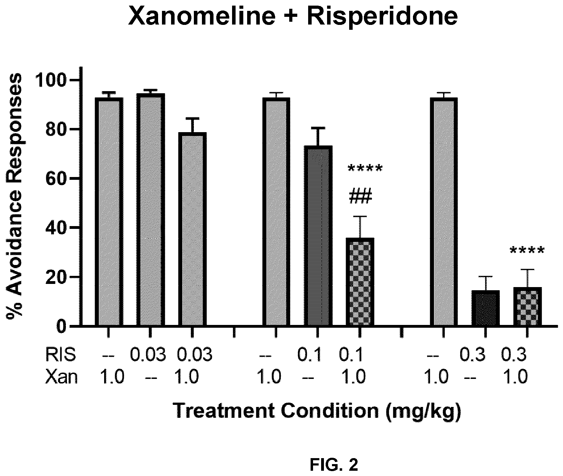

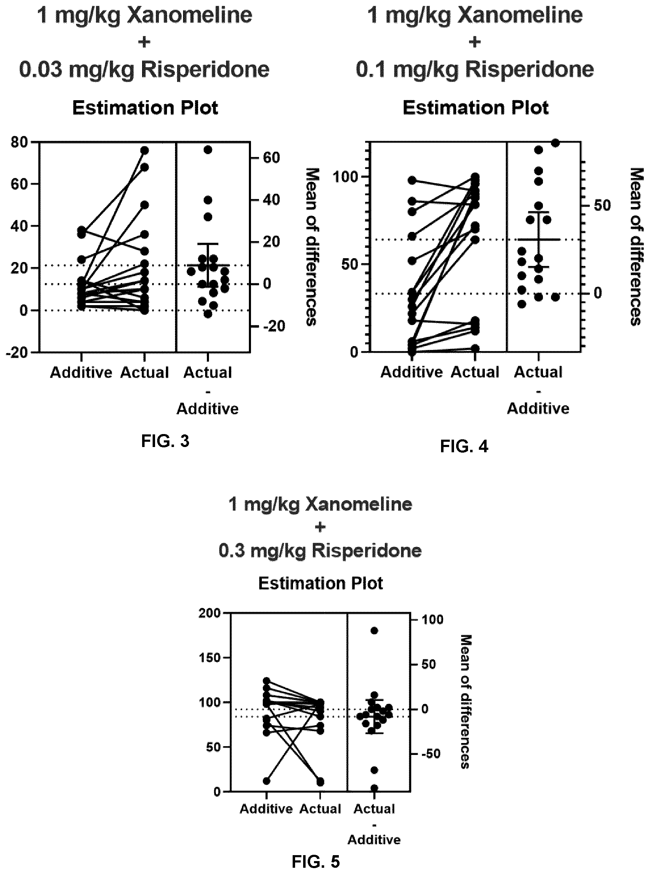

FIG. 2 shows the effects of a combination of low dose risperidone (RIS) with subthreshold xanomeline (Xan) on avoidance response [F(3.80,60.74) = 56.64, p < 0.001], N = 17. Data are planned comparisons vs. xanomeline alone (^{∗}) and risperidone dose alone (#). Table 6 shows that actual avoidance responses were significantly higher than expected if the effects of xanomeline alone and risperidone alone were added together. These comparative data are also displayed at FIGS. 3-5. Additivity versus synergy could not be determined with the 1 mg/kg xanomeline + 0.3 mg/kg risperidone combination because of floor effects *(i.e.,* near-maximal efficacy achieved). However, significant motor side-effects *(i.e.,* escape failures) were not observed despite near-maximal efficacy.

**Table 6 - Average avoidance response percentages for xanomeline and risperidone combination therapy.**

| **Treatment** | **X alone** | **R alone** | **Additive** | **Actual** | **Δ** | **Significance** |
|---|---|---|---|---|---|---|
| X at 1 mg/kg + R at 0.03 mg/kg | 7% | 5% | 12% | 21% | 9 | p=.08 |
| X at 1 mg/kg + R at 0.1 mg/kg | 7% | 26% | 33% | 64% | 31 | **p=.007** |
| X at 1 mg/kg + R at 0.3 mg/kg | 7% | 85% | 92% | 84% | -8 | p=.36 |

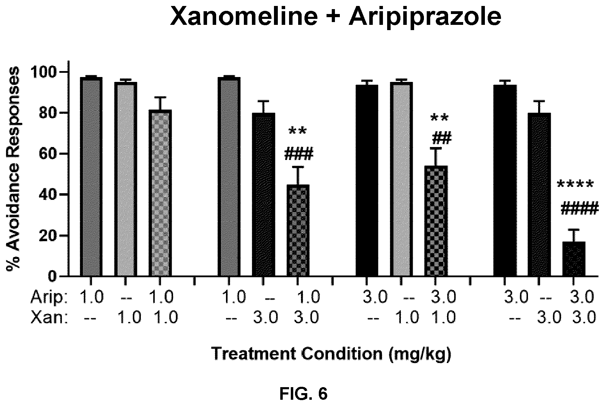

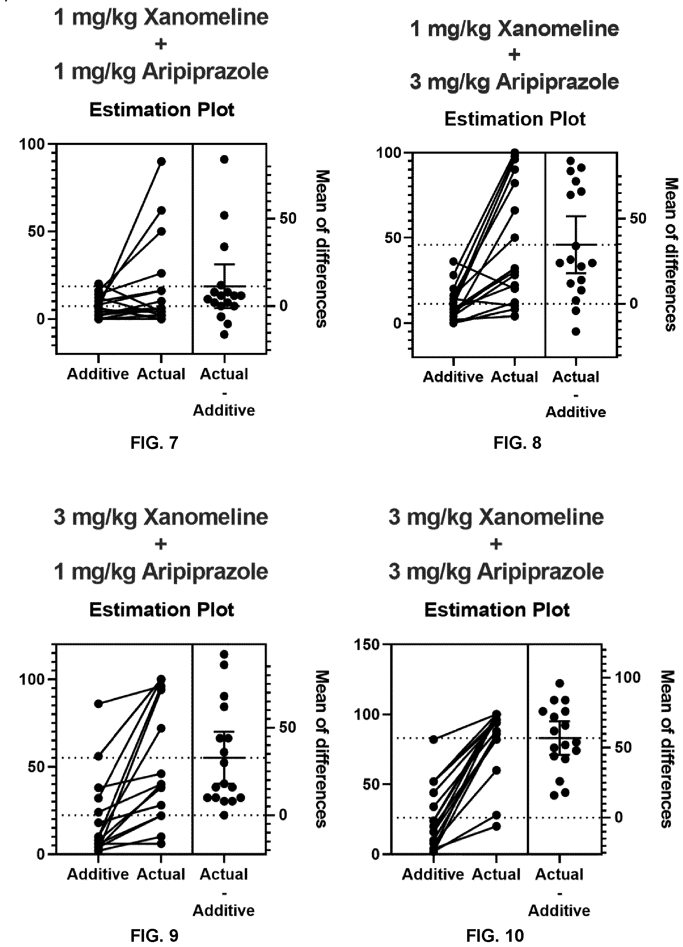

FIG. 6 shows the effects of subthreshold doses of aripiprazole (Arip) and xanomeline on avoidance responding [F(3.32,53.07) = 39.54, p < 0.0001], N = 17. Administration of either risperidone or aripiprazole alone did not significantly differ from vehicle-vehicle control conditions (data not shown). No incidence of escape failures was significant across any treatment condition. Post hoc analysis vs. xanomeline alone (^{∗}) and vs. comparator dose alone (#). Values are mean ± SEM. ^{∗∗} p<0.01, ^{∗∗∗∗} p<0.0001; ## p<0.01, ### p<0.001, #### p<0.0001. Table 7 shows that actual avoidance responses were significantly higher than expected if the effects of xanomeline alone and aripiprazole alone were added together. These comparative data are also displayed at FIGS. 7-10.

**Table 7 - Average avoidance response percentages for xanomeline and aripiprazole combination therapy.**

| **Treatment** | **X alone** | **A alone** | **Additive** | **Actual** | **Δ** | **Significance** |
|---|---|---|---|---|---|---|
| X at 1 mg/kg + A at 1 mg/kg | 5% | 2% | 7% | 19% | 12 | p=.07 |
| X at 1 mg/kg + A at 3 mg/kg | 5% | 6% | 11% | 46% | 35 | **p=.0004** |
| X at 3 mg/kg + A at 1 mg/kg | 20% | 2% | 22% | 55% | 33 | **p=.0002** |
| X at 3 mg/kg + A at 3 mg/kg | 20% | 6% | 26% | 83% | 57 | **p<.0001** |

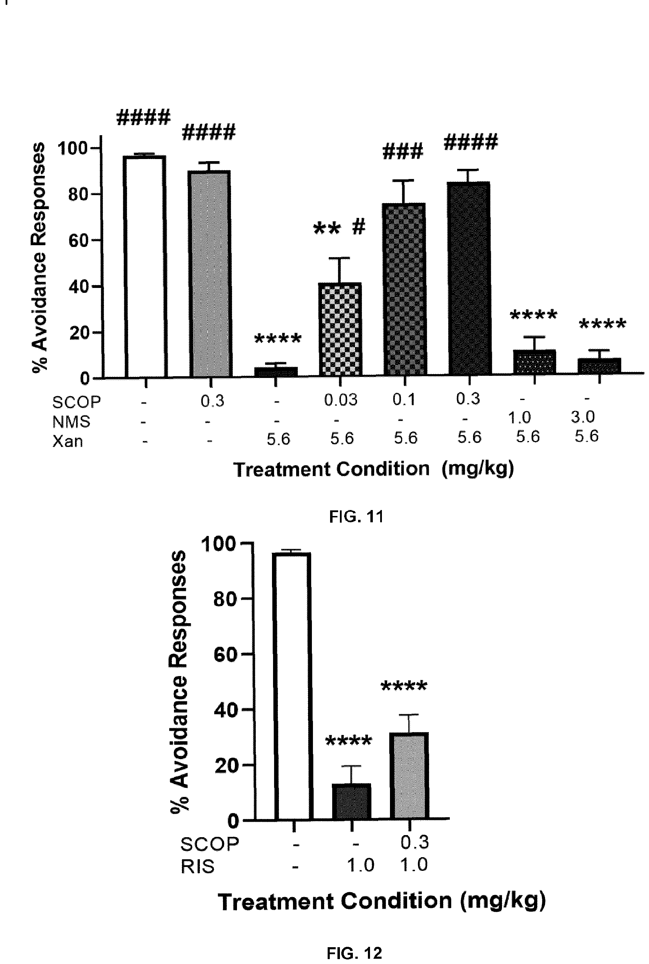

Xanomeline's antipsychotic effect in CAR depended on central muscarinic receptor activity. FIG. 11 shows xanomeline's (Xan) effect following blockade of central and peripheral muscarinic receptors by scopolamine (SCOP) and by peripheral-only muscarinic receptors by N-methyl-scopolamine (NMS). FIG. 11 confirms that xanomeline depends on central muscarinic receptors. SCOP dose-dependently blocked xanomeline's effects, but NMS had no effect. A maximally active dose of xanomeline was used in this study. The 0.3 mg/kg dose of SCOP that fully blocks the activity of xanomeline has no activity alone in CAR. SCOP partially blocks the activity of xanomeline at 0.03 mg/kg and fully at 0.1 mg/kg. A 100-fold higher dose of the peripherally-restricted muscarinic antagonist *N*-methyl-scopolamine failed to block the activity of xanomeline. [F(2.93, 38.15) = 58.09, p<0.0001], N = 14.

Referring to FIG. 12, a dose of scopolamine (0.3 mg/kg) that fully reversed xanomeline activity failed to attenuate risperidone's (RIS) antipsychotic-like effect in CAR [F(1.55,23.22) = 78.82, p<0.0001], N = 16. No incidence of escape failures were significant across any treatment condition. Values are mean ± SEM. Post hoc comparisons ^{∗∗} p<0.01, ^{∗∗∗∗} p<0.0001 vs. Veh/Veh; # p<0.05, ### p<0.001, #### p<0.0001 vs. Veh/Xan. FIG. 12 shows that risperidone does not depend on central muscarinic receptors for its activity. Xanomeline and risperidone differ mechanistically from each other. Including trospium is not expected to affect xanomeline's therapeutic activity.

NMS is an anticholinergic agent that lacks or has minimal penetration in the brain, thus blocking unwanted peripheral muscarinic activation from xanomeline, but allowing xanomeline to activate central muscarinic receptors. Suitable examples of other muscarinic antagonists include, but are not limited to, trospium, tolterodine, darifenacin, solifenacin, fesoterodine, and a salt thereof. These other muscarinic antagonists can be used in *in-vitro* or animal models or human clinical investigations with xanomeline and a composition comprising an antipsychotic.

In the LMA assay, varying doses of xanomeline and risperidone were co-formulated. They were dosed 30 minutes before a challenge dose of the N-methyl-D-aspartate (NMDA) receptor antagonist MK-801, followed by an evaluation of open-field activity for 60 minutes. After the experiment, plasma and brain tissue were collected to determine drug exposure.

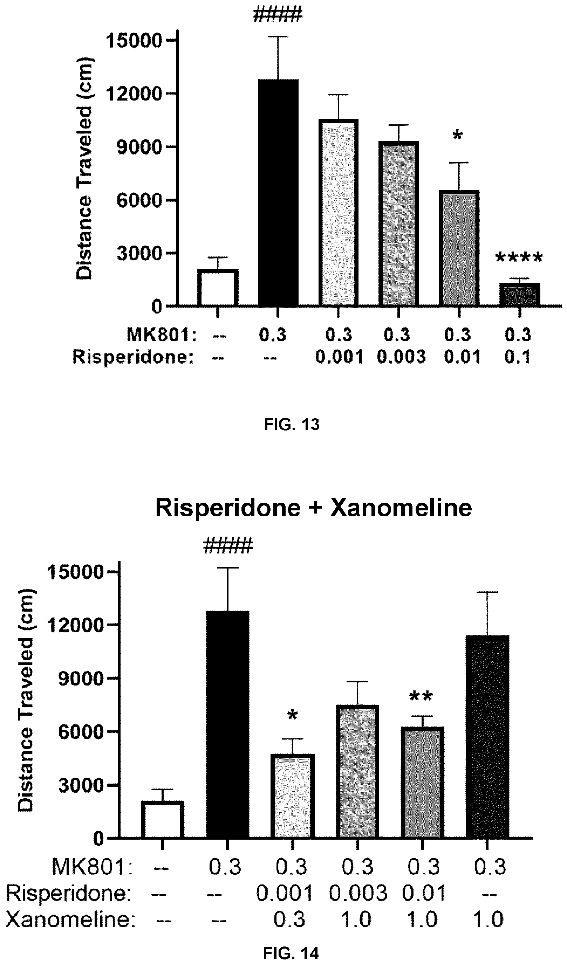

Xanomeline synergizes a sub-efficacious dose of risperidone following the MK-801 challenge in a locomotor assay. In a large multi-dose combination study with xanomeline and risperidone, FIG. 13 and 14 depict the effects of risperidone doses alone against MK-801 induced activity (FIG 13) and when combined with xanomeline (FIG 14). Inactive doses of xanomeline (1 mg/kg) and risperidone (0.001 mg/kg) when combined resulted in almost a 70% reduction in MK-801 stimulated activity. Data are mean (± SEM) total distance traveled. One-way ANOVAF(9,90)=7.34, p<0.001. #### significantly different from vehicle, p<0.0001; ^{∗} significantly different from MK-801/veh, p<0.05; ^{∗∗} significantly different from MK-801/veh, p<0.01; ^{∗∗∗∗} significantly different from MK-801/veh, p<0.0001.

Pharmacokinetic interactions do not explain the synergistic effects of xanomeline and risperidone in reducing MK-801-induced activity. Referring to Table 8, brain levels of xanomeline, risperidone, and MK-801 were collected immediately after the locomotor study in FIGS. 13 and 14. Plasma levels of combination compounds did not differ from drug-alone groups. Comparing 1.0 mg/kg xanomeline alone with xanomeline + risperidone dose groups revealed a significant effect via one-way between-groups ANOVA [F(3,36) = 6.25, p = 0.0016] with the 0.1 mg/kg risperidone + xanomeline differing from 1.0 mg/kg xanomeline alone (Dunnett test; ^{∗}p<0.05).

However, the 0.001 mg/kg risperidone + xanomeline group exhibited an augmented blockade of activity compared to the brain levels of xanomeline alone. MK-801 brain levels in the 0.1 mg/kg risperidone group were elevated compared to MK-801 alone, but MK-801 was unaffected in the risperidone + xanomeline groups. Exposures of risperidone or xanomeline alone groups were not measured. BLD = below level of detection; 0.2 ng/mL for risperidone and MK-801; 0.4 ng/mL for xanomeline. Technical limitations prevented measuring risperidone at the active doses.

**Table 8**

| **Treatment (mg/kg)** | | | | **Brain (ng/g)** | | |
|---|---|---|---|---|---|---|
| **MK-801** | **Risperidone** | **Xanomeline** | | **MK-801** | **Risperidone** | **Xanomeline** |
| 0.3 | | | | 386.9 ± 20.7 | | |
| 0.3 | | 1 | | 373.5 ± 12.6 | | 126.0 ± 6.6 |
| 0.3 | 0.001 | | | 381.9 ± 21.3 | BLD | |
| 0.3 | 0.001 | 1 | | 409.3 ± 23.5 | BLD | 143.4 ± 4.8 |
| 0.3 | 0.003 | | | 394.7 ± 19.0 | BLD | |
| 0.3 | 0.003 | 1 | | 405.7 ± 18.2 | BLD | 154.6 ± 9.3 |
| 0.3 | 0.01 | | | 416.6 ± 20.5 | BLD | |
| 0.3 | 0.01 | 1 | | 424.1 ± 26.8 | BLD | 184.9 ± 15.5^{∗} |
| 0.3 | 0.1 | | | 512.7 ± 28.7^{∗} | 22.9 ± 3.0 | |

There is no significant increase of xanomeline plasma exposure across the increasing doses of risperidone. Moreover, at the 0.01 mg/kg risperidone dose, xanomeline does not alter risperidone plasma levels. Thus, no significant drug-drug interaction effects were observed.

### Example 2 - Aripiprazole formulation increased response alone in the Mouse CAR Assay

Example 1 used a co-formulation of xanomeline and aripiprazole to deliver a single subcutaneous injection. This formulation limited aripiprazole exposure levels. This Example 2 used a formulation that enhanced aripiprazole absorption, resulting in a more robust response for aripiprazole alone. Also, in Example 2, compounds were delivered as two sequential subcutaneous injections rather than one. As a result, aripiprazole was evaluated at 10-fold lower doses in Example 2 than used in Example 1.

In Example 2, a 5% vehicle solution was prepared from 1.0 mL Tween-80 dissolved in 19.0 mL saline. To aid the solubility of the aripiprazole, three drops of 1.0-N HCl (15 µL) were added and vortexed. The average pH was 3.8 and compound was dosed at a 10 mL/kg bodyweight volume. Drug solutions were prepared fresh on each dosing day before testing. All solutions were mixed or vortexed thoroughly before injections.

Subjects (N=25) were male C57BL/6J, purchased from Jackson Labs, and successfully trained for CAR. Mice were first tested in an aripiprazole dose response study comprised of VEH, 0.3, 1, 3, and 10 mg/kg (data not shown). Dose order was randomly assigned for each mouse. Mice were returned to their home cages for 30 minutes following injection to allow the drug to take effect before testing began.

Aripiprazole was effective at disrupting CAR performance in a dose-related manner. The 0.3 mg/kg dose best approximated the ED₅₀. Escape failures were not evident at any doses.

To match the formulation procedure from Example 1 while maintaining an appropriate volume, a relatively high concentration aripiprazole stock was first made and then diluted to the target concentrations for dosing. A 5% Tween-80 solution was prepared from 2.0 mL Tween-80 dissolved in 38.0 mL of saline. A 3.0 mg/kg aripiprazole solution as a stock was prepared from 3.0 mg aripiprazole dissolved in 5.0 mL 5% Tween-80, giving a concentration of 0.6 mg/mL. At a dosing volume of 5 mL/kg, equaling 3.0 mg/kg. To aid the solubility of the aripiprazole, three drops of 1.0-N HCl (15 µL) were added and vortexed. The average pH was 6.2. For 0.3 mg/kg aripiprazole, 0.6 mL of the 3 mg/kg aripiprazole stock was diluted in 5.4 mL of 5% Tween-80. For 0.1 mg/kg aripiprazole, 0.2 mL of the 3 mg/kg aripiprazole stock was dissolved in 5.8 mL of 5% Tween-80.

The 1.0 mg/kg xanomeline solution was prepared from 3.05 mg xanomeline dissolved in 10.0 mL of sterile water, giving a concentration of 0.2 mg/mL. At a dosing volume of 10 mL/kg, this equals 1.0 mg/kg. (The drug mass adjustment factor between xanomeline free base and xanomeline tartrate is 1.5262.) The average pH was 4.4.

Mice were tested in CAR until all mice had completed the dosing regimen (see Table 9 below). Dose order was randomly assigned for each mouse. Injection 1 and Injection 2 occurred in sequence, with no gap time. Mice were returned to their home cages for 30 minutes following injection to allow the drugs to take effect before testing began. Mice were injected with vehicle (saline at 10 mL/kg SC and Tween-80 at 5 mL/kg IP) the day before dosing days. Mice were only dosed if their average percent avoidance values for the previous two non-dose days was > 85%.

**Table 9 - Treatment Groups and Dosing Paradigm**

| **Group** | **Treatment** | **Injection 1 (5 mL/kg IP)** | **Injection 2 (10 mL/kg SC)** |
|---|---|---|---|
| 1 | Vehicle | 5% Tween-80 solution | Saline |
| 2 | 1.0 mg/kg xanomeline | 5% Tween-80 solution | 1.0 mg/kg xanomeline |
| 3 | 0.1 mg/kg aripiprazole | 0.1 mg/kg aripiprazole | Saline |
| 4 | 0.3 mg/kg aripiprazole | 0.3 mg/kg aripiprazole | Saline |
| 5 | 0.1 mg/kg aripiprazole + 1.0 mg/kg xanomeline | 0.1 mg/kg aripiprazole | 1.0 mg/kg xanomeline |
| 6 | 0.3 mg/kg aripiprazole + 1.0 mg/kg xanomeline | aripiprazole | 1.0 mg/kg xanomeline |

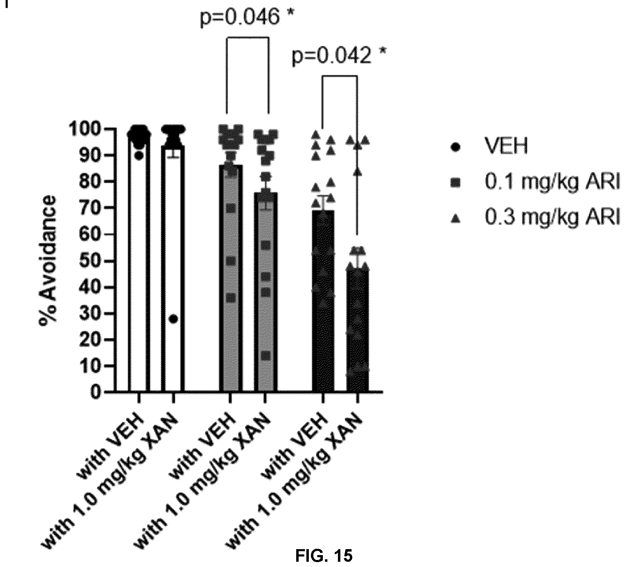

Referring to FIG. 15, the percent avoidance was analyzed via a two-way repeated-measures ANOVA with aripiprazole dose (vehicle, 0.1, or 0.3 and xanomeline dose (vehicle or 1.0) as factors. Planned comparisons of with or without xanomeline at the vehicle, 0.1, and 0.3 mg/kg aripiprazole (Bonferroni multiple comparisons corrected); ^{∗} p<.05 versus without xanomeline. Main Effect Xano Tx: F(1,15)=11.00, p=.0047 / Main Effect Aripiprazole Tx: F(1.817, 27.26)=26.01, p<.0001 / Interaction: F(1.526,22.89)=3.074, p=.0774. N=16. VEH=vehicle; XAN=xanomeline; ARI=aripiprazole.

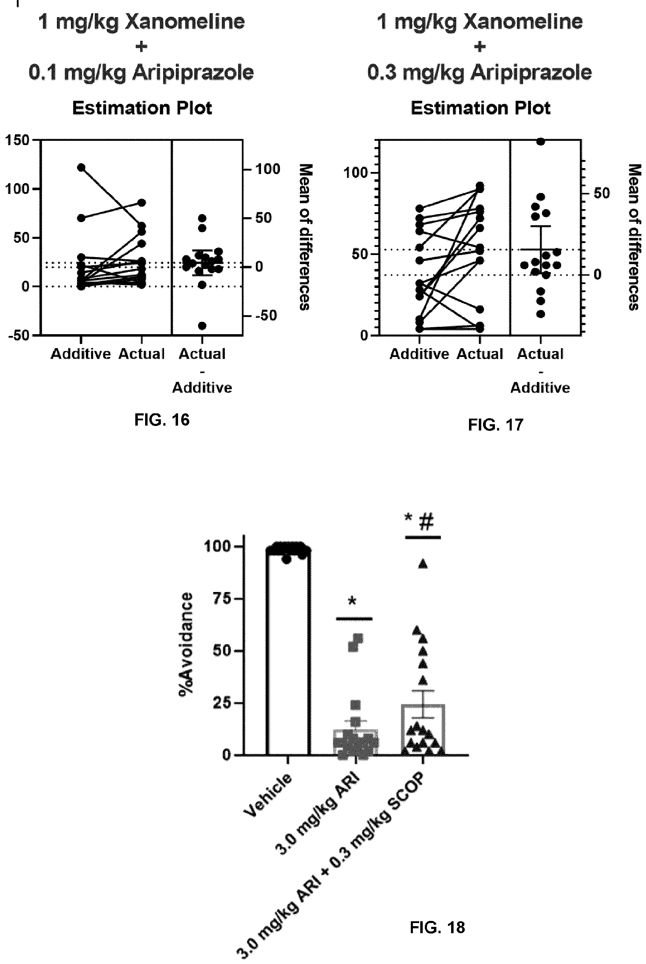

Table 10 shows that actual avoidance responses were higher than what would be expected if the effects of xanomeline alone and aripiprazole alone were added together. These comparative data are also displayed at FIGS. 16 & 17.

**Table 10 - Average avoidance response percentages for xanomeline and aripiprazole combination therapy.**

| **Treatment** | **X alone** | **A alone** | **Additive** | **Actual** | **Δ** | **Significance** |
|---|---|---|---|---|---|---|
| X at 1 mg/kg + A at 0.1 mg/kg | 6% | 14% | 20% | 24% | 4 | p=.47 |
| X at 1 mg/kg + A at 0.3 mg/kg | 6% | 31% | 37% | 53% | 16 | **p=.036** |

Under these study conditions, the percentage avoidance responses and latency to respond evinced that a 1 mg/kg dose was inactive in the CAR assay. However, xanomeline significantly augmented the activity of 0.1 and 0.3 mg/kg aripiprazole in both endpoints. No dose of aripiprazole or combination dose with xanomeline resulted in escape failures.

The study then evaluated the effect of blocking muscarinic receptors (via scopolamine) on the activity and efficacy of aripiprazole in the Conditioned Avoidance Response (CAR) assay. The aripiprazole solution was prepared as before. To prepare the 0.3 mg/kg scopolamine solution, 3.397 mg scopolamine was dissolved in 40 mL of saline. The actual drug weight of SCOP in this solution is 2.4 mg (adjusted by a factor 1.41499 for the salt content), giving a concentration of 0.06 mg/mL. At a dosing volume of 5 mL/kg, this equals 0.3 mg/kg. The average pH of this solution was 7.3.

Mice were tested in CAR until all mice had completed the dosing regimen (Table 11). Dose order was randomly assigned for each mouse. Injection 1 was administered 15 minutes before Injection 2. During the interim, mice were returned to their home cages for 30 minutes following the final injection to allow the drugs to take effect before testing began. Mice were injected with vehicle (saline at 5 mL/kg S.C. and Tween-80 at 5 mL/kg I.P.) the day before dosing days. Mice were only dosed if their average percent avoidance values for the previous two non-dose days was > 85%.

**Table 11 - Treatment Groups and Dosing Paradigm**

| **Group** | **Treatment** | **Injection 1 (5 mL/kg S.C.)** | **Injection 2 (5 mL/kg I.P.)** |
|---|---|---|---|
| 1 | Vehicle | Saline | 5% Tween-80 solution |
| 2 | 3.0 mg/kg aripiprazole | Saline | 3.0 mg/kg aripiprazole |
| 3 | 0.3 mg/kg scopolamine + 3.0 mg/kg aripiprazole | 0.3 mg/kg scopolamine | 3.0 mg/kg aripiprazole |

Referring to FIG. 18, avoidance behavior was analyzed via a one-way repeated measures ANOVA. We found a significant effect of drug treatment, with Tukey post-hoc analysis showing differences in all comparisons. F(1.35,21.64)=176.3, p<.0001. ^{∗}p<.0001 vs. Vehicle; #p=.02 versus Aripiprazole alone. N=15. Ari=aripiprazole; SCOP=scopolamine.

A 3 mg/kg dose of aripiprazole had robust effects on CAR activity endpoints. Scopolamine had a slight, but statistically significant effect in reducing the activity of aripiprazole. However, robust activity of the scopolamine + aripiprazole combination was observed relative to vehicle control.

Consistent with preclinical and clinical reports, xanomeline exhibited antipsychotic-like activity. Combined low doses of xanomeline with risperidone and aripiprazole significantly augmented the CAR and LMA effects (risperidone only tested) over those observed for each agent alone and did not appear to drive the motor adverse effects commonly observed with risperidone and aripiprazole. This result indicates synergy between the xanomeline and the other two compounds. Central muscarinic receptors mediated xanomeline's efficacy in these animal models of psychosis.

This therapeutic synergy was not attributable to increased compound exposures due to drug-drug interactions. It did not appear to drive motor side-effects commonly observed with high doses of antipsychotic agents. These data support investigating synergy in clinical trials to determine the therapeutic benefit of KarXT when combined with traditional antipsychotic agents. Given the established role of muscarinic (M1/M4) receptors in cognitive processes, future studies to assess the role of xanomeline in preclinical models of negative symptoms and cognitive deficits associated with schizophrenia.

The preceding description is given for clearness of understanding only. No unnecessary limitations should be understood from there, as modifications within the disclosure scope may be apparent to those having ordinary skill in the art. Throughout the specification, where compositions are described as including components or materials, it is contemplated that the compositions can also consist essentially of, or consist of, any combination of the recited components or materials, unless described otherwise. Likewise, where methods are described as including steps, it is contemplated that the methods can also consist essentially of, or consist of, any combination of the recited steps, unless described otherwise. The disclosure illustratively disclosed herein suitably may be practiced in the absence of any element or step not specifically disclosed herein.

The practice of a method disclosed herein, and individual steps thereof, can be performed manually and/or with the aid of or automation provided by electronic equipment. Although processes have been described regarding embodiments, a person of ordinary skill in the art will readily appreciate that other ways of performing the acts associated with the methods may be used. For example, the order of various steps may be changed without departing from the scope or spirit of the method unless described otherwise. Some of the individual steps can also be combined, omitted, or further subdivided into additional steps.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination. All combinations of the embodiments pertaining to the chemical groups represented by the variables contained within the generic chemical formulae described herein are specifically embraced by the present invention just as if each combination was individually explicitly recited, to the extent that such combinations embrace stable compounds (i.e., compounds that can be isolated, characterized and tested for biological activity). Also, all subcombinations of the chemical groups listed in the embodiments describing such variables, as well as all subcombinations of uses and medical indications described herein, are also specifically embraced by the present invention just as if each and every subcombination of chemical groups and subcombination of uses and medical indications was individually and explicitly recited herein.

All patents, publications and references cited herein are hereby fully incorporated by reference. In case of conflict between the present disclosure and incorporated patents, publications and references, the present disclosure should control.

## Claims

1. A method of treating a central nervous system disorder in a patient in need thereof, the method comprising co-administering a first composition comprising xanomeline and/or a salt thereof and a composition comprising an antipsychotic.

2. The method of claim 1, wherein the first composition or the second composition or both are co-administered in a reduced amount compared to the first composition or the second composition used alone.

3. The method of claim 1 or 2, wherein the disorder is chosen from dementia-related psychosis, schizophrenia, Alzheimer's disease, Parkinson's disease, depression, movement disorders, pain, drug addiction, tauopathy, and synucleinopathy.

4. The method of any one of claims 1-3, wherein the administration is oral.

5. The method of any one of claims 1-4, wherein the antipsychotic is an atypical antipsychotic.

6. The method of claim 5, wherein the atypical antipsychotic is chosen from risperidone, olanzapine, clozapine, quetiapine, aripiprazole, and ziprasidone.

7. The method of claim 5, wherein the atypical antipsychotic is risperidone or aripiprazole.

8. The method of any one of claims 1-7, wherein the xanomeline and/or a salt thereof is xanomeline tartrate.

9. The method of any one of claims 1-8, wherein the composition comprising xanomeline and/or a salt thereof further comprises an anticholinergic agent.

10. The method of claim 9, wherein the anticholinergic agent is a muscarinic antagonist.

11. The method of claim 10, wherein the muscarinic antagonist is chosen from trospium, tolterodine, darifenacin, solifenacin, fesoterodine, scopolamine, *N*-methylscopolamine, and a salt thereof.

12. The method of claim 11, wherein the muscarinic antagonist is trospium chloride.

13. A method of treating a central nervous system disorder in a patient in need thereof, the method comprising co-administering of a first composition comprising xanomeline and/or a salt thereof and trospium chloride, and a second composition comprising an antipsychotic.

14. The method of claim 13, wherein the first composition or the second composition or both are co-administered in a reduced amount compared to the first composition or the second composition used alone.

15. The method of claim 13 or 14, wherein the disorder is chosen from dementia-related psychosis, schizophrenia, Alzheimer's disease, Parkinson's disease, depression, movement disorders, pain, drug addiction, tauopathy, and synucleinopathy.

16. The method of any one of claims 14-15, wherein the administration is oral.

17. The method of any one of claims 12-16, wherein the antipsychotic is an atypical antipsychotic.

18. The method of claim 17, wherein the atypical antipsychotic is chosen from risperidone, olanzapine, clozapine, quetiapine, aripiprazole, and ziprasidone.

19. The method of claim 18, wherein the atypical antipsychotic is risperidone or aripiprazole.

20. The method of any one of claims 14-19, wherein the xanomeline and/or a salt thereof is xanomeline tartrate.

21. The method of any one of claims 14-20, wherein the xanomeline and/or a salt thereof and salt of trospium are co-administered as a pharmaceutical composition comprising a plurality of xanomeline beads having a core comprising the xanomeline or a salt thereof, and a plurality of trospium beads having a core comprising the salt of trospium.

22. The method of any one of claims 14-20, wherein the pharmaceutical composition is a capsule containing the plurality of xanomeline beads and the plurality of trospium beads.

23. The method of any one of claims 14-22, wherein total daily dose is chosen from 5 mg a salt of trospium,
10 mg a salt of trospium,
15 mg a salt of trospium,
17.5 mg a salt of trospium,
20 mg a salt of trospium,
30 mg a salt of trospium,
25 mg xanomeline and/or the salt thereof,
50 mg xanomeline and/or the salt thereof,
75 mg xanomeline and/or the salt thereof,
100 mg xanomeline and/or the salt thereof,
150 mg xanomeline and/or the salt thereof,
175 mg xanomeline and/or the salt thereof,
200 mg xanomeline and/or the salt thereof,
25 mg xanomeline and/or the salt thereof and 5 mg a salt of trospium,
50 mg xanomeline and/or the salt thereof and 10 mg a salt of trospium,
50 mg xanomeline and/or the salt thereof and 20 mg a salt of trospium,
75 mg xanomeline and/or the salt thereof and 15 mg a salt of trospium,
75 mg xanomeline and/or the salt thereof and 30 mg a salt of trospium,
100 mg xanomeline and/or the salt thereof and 17.5 mg a salt of trospium,
100 mg xanomeline and/or the salt thereof and 20 mg a salt of trospium,
150 mg xanomeline and/or the salt thereof and 20 mg a salt of trospium,
150 mg xanomeline and/or the salt thereof and 30 mg a salt of trospium,
175 mg xanomeline and/or the salt thereof and 30 mg a salt of trospium, and
200 mg xanomeline and/or the salt thereof and 30 mg a salt of trospium.

24. The method of any one of claims 14-22, wherein the total daily dose is between 100 and 200 mg xanomeline and/or a salt thereof and between 10 and 30 mg of a salt of trospium.

25. The method of claim 24, wherein the total daily dose is between 100 and 200 mg xanomeline and/or a salt thereof and between 20 and 30 mg of a salt of trospium.

26. The method of claim 25, wherein the total daily dose is between 100 and 200 mg xanomeline and/or a salt thereof and about 30 mg of a salt of trospium.

27. The method of claim 26, wherein the total daily dose is about 200 mg xanomeline and/or a salt thereof and about 30 mg of a salt of trospium.

28. The method of any one of claims 24-27, wherein the total daily dose is co-administered in two or three intervals.

29. The method of any one of claims 1-28, wherein the patient is treated for at least 7 days.

30. The method of any one of claims 1-29 via a titration scheme that comprises down-titration of the antipsychotic.

31. The method of any one of claims 1-30 via a titration scheme that comprises down-titration of the xanomeline, or a salt thereof, and the salt of trospium.

32. A method of treating schizophrenia in a patient in need thereof, the method comprising co-administering a combination of a composition comprising xanomeline and/or a salt thereof and a composition comprising risperidone or aripiprazole.

33. The method of claim 32, wherein the first composition or the second composition or both are co-administered in a reduced amount compared to the first composition or the second composition used alone.

34. The method of claim 32 or 33, wherein the administration is oral.

35. The method of any one of claims 32-34, wherein the xanomeline and/or a salt thereof is xanomeline tartrate.

36. The method of any one of claims 32-35, wherein the composition comprising xanomeline and/or a salt thereof further comprises a salt of trospium.

37. The method of claim 36, wherein the salt of trospium is trospium chloride.

38. The method of claim 36 or 37, wherein the xanomeline and/or a salt thereof and salt of trospium are co-administered as a pharmaceutical composition comprising a plurality of xanomeline beads having a core comprising the xanomeline or a salt thereof, and a plurality of trospium beads having a core comprising the salt of trospium.

39. The method of claim 38, wherein the pharmaceutical composition is a capsule containing the plurality of xanomeline beads and the plurality of trospium beads.

40. The method of any one of claims 32-39 via a titration scheme that comprises down-titration of the antipsychotic.

41. The method of any one of claims 32-40 via a titration scheme that comprises down-titration of the xanomeline, or a salt thereof, and the salt of trospium.
